(19) **Europäisches Patentamt / European Patent Office / Office européen des brevets**

(11) **EP 1 764 084 A1**

(12) **DEMANDE DE BREVET EUROPEEN**

(43) Date de publication:
**21.03.2007 Bulletin 2007/12**

(51) Int Cl.:
*A61K 8/86* *(2006.01)*   *A61Q 1/14* *(2006.01)*

(21) Numéro de dépôt: **06119306.6**

(22) Date de dépôt: **22.08.2006**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **20.09.2005 FR 0552821**

(71) Demandeur: **L'ORÉAL**
**75008 Paris (FR)**

(72) Inventeur: **Bonafos, Arnaud**
**75014 Paris (FR)**

(74) Mandataire: **Rasson, Catherine**
**L'OREAL - D.I.P.I.**
**25-29 Quai Aulagnier**
**92600 Asnières (FR)**

Remarques:
Revendications modifiées conformément à la règle 86 (2) CBE.

(54) **Composition démaquillante**

(57) L'invention a pour objet une composition cosmétique aqueuse démaquillante, caractérisée en ce qu'elle contient un dérivé de polyéthylène glycol et d'un mélange de glycérides d'acide caprylique et d'acide caprique, comportant 7 groupes d'oxyde d'éthylène, en une quantité inférieure à 10 % en poids par rapport au poids total de la composition.

La composition selon l'invention peut constituer notamment une eau démaquillante ayant un bon confort oculaire, utilisée notamment pour le démaquillage en douceur de la peau et/ou de la zone oculaire.

**Description**

L'invention a pour objet une composition cosmétique aqueuse, contenant un tensioactif particulier, le PEG- 7 Caprylic/Capric Glycerides, et l'utilisation de la dite composition pour le démaquillage de la peau et/ou des yeux. L'invention a aussi pour objet l'utilisation cosmétique de ce tensioactif particulier pour le démaquillage des yeux et/ou de la peau.

La personne utilisant des mascaras, des fonds de teint ou des rouges à lèvres, notamment des produits de longue tenue et « waterproof », est à la recherche de produits permettant un bon démaquillage, avec un confort d'utilisation optimal, c'est-à-dire ayant aussi bien de bonnes qualités cosmétiques qu'une bonne tolérance. Elle cherche donc à avoir un produit de démaquillage qui ait une bonne efficacité de démaquillage tout en étant doux, agréable à utiliser et bien toléré, c'est-à-dire ne produisant pas d'irritations.

Les produits couramment proposés contiennent souvent une quantité importante d'huile, la présence d'huile permettant d'obtenir une bonne efficacité de démaquillage. Cependant, ces produits ont l'inconvénient de laisser un film gras désagréable et inesthétique sur la peau. Par ailleurs, il existe des compositions aqueuses démaquillantes qui contiennent des tensioactifs et qui sont exemptes d'huile, mais dont l'efficacité sur le maquillage de longue tenue est limitée et souvent insuffisante.

Il subsiste donc le besoin de disposer d'une composition aqueuse de démaquillage, ayant un pouvoir démaquillant amélioré, tout en étant très confortable pour la peau et les yeux et tout en ayant donc une bonne tolérance et notamment une bonne tolérance oculaire, la zone oculaire étant particulièrement sensible.

Or, la demanderesse a découvert de manière surprenante que l'addition d'un dérivé de polyéthylène glycol et d'un mélange de glycérides (mono-, di- et tri-glycérides) d'acide caprylique et d'acide caprique comportant 7 groupes d'oxyde d'éthylène (nom CTFA : PEG-7 caprylic/capric glycerides) en une quantité limitée permettait de résoudre ce problème et d'obtenir des compositions aqueuses dépourvues d'huiles, ayant une très bonne efficacité démaquillante, notamment sur les fonds de teint de longue tenue, en comparaison à des tensioactifs couramment utilisés dans le démaquillage.

La présente invention a donc pour objet une composition cosmétique aqueuse démaquillante, caractérisée par le fait qu'elle contient un dérivé de polyéthylène glycol et d'un mélange de glycérides d'acide caprylique et d'acide caprique, comportant 7 groupes d'oxyde d'éthylène, en une quantité inférieure à 10 % en poids par rapport au poids total de la composition.

Cette composition est exempte d'huile. En effet, on entend ici par « composition aqueuse », une composition exempte d'huile. La composition aqueuse de l'invention contient généralement au moins 55 % en poids d'eau, de préférence au moins 65 % en poids d'eau et encore mieux au moins 75 % en poids d'eau par rapport au poids total de la composition. La quantité d'eau peut aller notamment de 55 à 99 % en poids, de préférence de 65 à 98 % et mieux de 75 à 98 % en poids par rapport au poids total de la composition. La composition obtenue est généralement limpide, c'est-à-dire transparente à translucide. Sa transparence peut se mesurer par exemple par un coefficient de transmittance à 600 nm allant de 10 à 90 % ou bien par une turbidité allant de 60 à 600 NTU et de préférence de 70 à 300 NTU, turbidité mesurée au turbidimètre portatif HACH - Modèle 2100 P.

La composition obtenue peut constituer notamment une eau démaquillante de type lotion ou un gel. Le mot « lotion » signifie que la composition est liquide et a une viscosité inférieure à 0,1 Pa.s (100 centipoises), de préférence inférieure à 0,06 Pa.s (60 centipoises), cette viscosité étant mesurée à 25°C avec un appareil Rheomat RM 180 de Rheometric Scientific, cet appareil étant équipé d'un mobile différent selon les viscosités, par exemple d'un mobile 1 ou 2 pour les gammes de viscosités inférieures à 7 poises, d'un mobile 3 pour les gammes de viscosités de 2 à 40 poises, et d'un mobile 4 pour les gammes de viscosités de 20 poises à 100 poises.

Un gel peut avoir une viscosité allant jusqu'à 5 Pa.s. La viscosité des gels selon l'invention va de préférence de 0,1 à 200 poises (0,01 à 20 Pa.s), et mieux de 5 à 150 poises (0,5 à 15 Pa.s), mesurée à 25°C à l'aide du Rheomat RM 180.

La composition selon l'invention est destinée à une application topique et contient donc un milieu physiologiquement acceptable. Par milieu physiologiquement acceptable, on entend un milieu compatible avec la peau, les muqueuses (y compris l'intérieur des paupières, les lèvres), les ongles, la zone oculaire et/ou les fibres kératiniques (cheveux et cils).

De manière inattendue, le dérivé de polyéthylène glycol et du mélange de glycérides d'acide caprylique et d'acide caprique, comportant 7 groupes d'oxyde d'éthylène, bien que présent en une quantité inférieure à 10 % et même de préférence en une quantité inférieure à 5 % du poids total de la composition, permet un bon démaquillage des matières kératiniques et notamment de la peau ou de la zone oculaire (cils, paupières). Elle permet par exemple une bonne élimination du mascara se trouvant sur les cils, une bonne élimination de l'eyeliner se trouvant sur les paupières et une bonne élimination du fond de teint se trouvant sur la peau.

Aussi, l'invention a encore pour objet l'utilisation cosmétique d'un dérivé de polyéthylène glycol et d'un mélange de glycérides d'acide caprylique et d'acide caprique, comportant 7 groupes d'oxyde d'éthylène, en une quantité inférieure à 10 % en poids par rapport au poids total de la composition, pour le démaquillage de la peau et de la zone oculaire.

Comme dérivé de polyéthylène glycol et d'un mélange de glycérides d'acide caprylique et d'acide caprique, comportant 7 groupes d'oxyde d'éthylène ou PEG-7 Caprylic/Capric Glycerides, on peut citer notamment le produit commercialisé sous la dénomination CETIOL HE 810 par la société Cognis.

La quantité de PEG-7 Caprylic/Capric Glycerides peut aller par exemple de 0,01 à 10 % en poids, de préférence de 0,05 à 10 % en poids, mieux de 0,05 à 5 % en poids et encore mieux de 0,5 à 5 % en poids par rapport au poids total de la composition.

En outre, la composition aqueuse de l'invention peut comprendre un ou plusieurs tensioactifs autres que le PEG-7 Caprylic/Capric Glycerides. La quantité totale de tensioactifs (PEG-7 Caprylic/Capric Glycerides + autres tensioactifs) va de préférence de 0,01 à 12 % en poids, plus préférentiellement 0,05 à 10% en poids, encore plus préférentiellement 2 à 10 % en poids et mieux de 3 à 10 % en poids par rapport au poids total de la composition.

On peut utiliser comme autre tensioactif, tout tensioactif habituellement utilisé dans les compositions démaquillantes. Le tensioactif peut être choisi parmi les tensioactifs non ioniques, les tensioactifs anioniques, les tensioactifs amphotères ou zwitterioniques et leurs mélanges.

On peut citer comme tensioactifs supplémentaires utilisables dans la composition selon l'invention :

(1) parmi les tensioactifs non ioniques :

- les polymères blocs oxyéthylénés oxypropylénés tels que les Poloxamers et notamment le Poloxamer 184 et le Poxamer 124 (nom CTFA) ;
- les esters d'acide gras de sorbitan et leurs dérivés oxyéthylénés, comme le monostéarate de sorbitan (nom CTFA : Sorbitan stearate) vendu par la société ICI sous la dénomination Span 60, le monopalmitate de sorbitan (nom CTFA : Sorbitan palmitate) vendu par la société ICI sous la dénomination Span 40, les stéarates, palmitates et oléates de sorbitan oxyéthylénés (nom CTFA : Polysorbate) vendus par la société ICI sous les dénominations Tween, notamment le Polysorbate 60 (Tween 60), le Polysorbate 65 (Tween 65), le Polysorbate 80 (Tween 80) ;
- les dérivés de polyéthylène glycol et d'un mélange de glycérides (mono-, di- et tri-glycérides) d'acide caprylique et d'acide caprique, ayant de 6 ou 8 groupes d'oxyde d'éthylène, tels que celui comportant 6 groupes d'oxyde d'éthylène (nom CTFA : PEG-6 caprylic/capric glycérides), commercialisé sous la dénomination SOFTIGEN 767 par la société Sasol, et celui comportant 8 groupes d'oxyde d'éthylène (nom CTFA : PEG-8 caprylic/capric glycérides), commercialisé sous la dénomination L.A.S. par la société Gattefosse ;
- les dérivés de polyéthylène glycol d'un mélange de glycérides (triglycerides alcoxylés) d'origine végétale, tels que par exemple PEG-10 Olive glycerides, PEG-45 Palm Kernel glycerides, PEG-20 Almond glycerides,
- les alkylpolyglycosides et notamment les alkylpolyglucosides (APG) ayant un groupe alkyle comportant de 6 à 30 atomes de carbone (alkyl-$C_6$-$C_{30}$ polyglucosides) et de préférence 8 à 16 atomes de carbone, comme par exemple le decylglucoside (Alkyl-C9/C11-polyglucoside (1.4) tel que le produit commercialisé sous la dénomination MYDOL 10 par la société Kao Chemicals, le produit commercialisé sous la dénomination PLANTAREN 2000 UP ou PLANTACARE 2000 UP par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ; le laurylglucoside comme les produits commercialisés sous les dénominations PLANTAREN 1200 N et PLANTACARE 1200 par la société Henkel ; et le coco-glucoside comme le produit commercialisé sous la dénomination PLANTACARE 818/UP par la société Henkel.

(2) parmi les tensioactifs anioniques :

- les alkylsulfates et leurs sels, les alkyl éther sulfates et leurs sels, notamment leurs sels de sodium, comme le mélange de Sodium Laureth Sulfate / Magnesium Laureth Sulfate / Sodium Laureth-8 Sulfate / Magnesium Laureth-8 Sulfate, vendu sous le nom de Texapon ASV par la société Henkel ; le lauryl éther sulfate de sodium (C12-14 70/30) (2,2 OE) commercialisé sous les dénominations SIPON AOS 225 ou Texapon N702 PATE par la société Henkel ; le lauryl éther sulfate d'ammonium (C12-14 70/30) (3 OE) commercialisé sous la dénomination SIPON LEA 370 par la société Henkel ; l'alkyl (C12-C14) éther (9 OE) sulfate d'ammonium commercialisé sous la dénomination RHODAPEX AB/20 par la société Rhodia Chimie.

(3) parmi les tensioactifs amphotères ou zwitterioniques, les alkylamphoacétates tels que le N-cocoyl-N-carboxyméthoxyéthyl-N-carboxyméthyl-éthylènediamine N-di-sodique (nom CTFA : Disodium cocoampho-diacetate) commercialisé en solution aqueuse saline sous la dénomination MIRANOL C2M CONC NP par la société Rhodia Chimie ; le N-cocoyl-N-hydroxyéthyl-N-carboxyméthyl-éthylènediamine N-sodique (nom CTFA : sodium cocamphoacetate) ; les cocamides tels que le mélange d'éthanolamides d'acide de coco (nom CTFA : Cocamide DEA).

Selon un mode préféré de réalisation de l'invention, le ou les tensioactifs supplémentaires sont choisis parmi les tensioactifs non ioniques, en particulier parmi les polymères blocs oxyéthylénés oxypropylénés, les esters de sorbitan polyoxyéthylénés, les dérivés de polyéthylène glycol d'un mélanges de glycérides d'acide caprylique et d'acide caprique, ayant de 6 ou 8 groupes d'oxyde d'éthylène, et leurs mélanges.

Selon un mode particulièrement préféré de réalisation de l'invention, la composition selon l'invention contient comme autre tensioactif au moins un dérivé de polyéthylène glycol d'un mélange de glycérides d'acide caprylique et d'acide caprique, ayant de 6 groupes d'oxyde d'éthylène. Cette association permet d'obtenir de très bons résultats de démaquillage avec une très bonne tolérance et un bon confort d'utilisation

Dans la composition de l'invention, l'eau utilisée peut être de l'eau pure déminéralisée mais aussi de l'eau minérale et/ou de l'eau thermale et/ou de l'eau de mer, c'est-à-dire que l'eau de la composition peut être en partie ou totalement constituée par une eau choisie parmi les eaux minérales, les eaux thermales, les eaux de mer et leurs mélanges. En général, une eau minérale est propre à la consommation, ce qui n'est pas toujours le cas d'une eau thermale. Chacune de ces eaux contient, entre autre, des minéraux solubilisés et/ou des oligo-éléments. Ces eaux sont connues pour être employées à des fins de traitement spécifique selon les oligo-éléments et les minéraux particuliers qu'elles contiennent, tel que l'hydratation et la désensibilisation de la peau ou le traitement de certaines dermatoses. Par eaux minérales ou thermales, on désignera non seulement les eaux minérales ou thermales naturelles, mais également des eaux minérales ou thermales naturelles enrichies en constituants minéraux et/ou en oligo-éléments supplémentaires, ainsi que des solutions aqueuses minérales et/ou contenant des oligo-éléments préparées à partir d'eau purifiée (déminéralisée ou distillée).

Une eau thermale ou minérale naturelle utilisée selon l'invention peut, par exemple, être choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avene.

On peut aussi utiliser des eaux de mer telles que l'eau de la mer Morte (Dead Sea water) ou l'eau des fonds marins.

La composition selon l'invention peut éventuellement contenir un alcool monohydrique en $C_1$-$C_3$, notamment d'éthanol. Selon un mode préféré de réalisation de l'invention, si la composition contient un tel alcool, la quantité d'alcool est inférieure à 5 % et de préférence inférieure à 3 % quand il s'agit d'un démaquillant pour les yeux.

La composition de l'invention peut comprendre un ou plusieurs polyols et notamment des glycols. Parmi les polyols utilisables dans la composition selon l'invention, on peut citer notamment le propylène glycol, le butylène glycol, la glycérine, l'hexylène glycol, les polyéthylène glycols tels que le PEG-8, le dipropylène glycol et leurs mélanges. La quantité de polyol(s) dans la composition de l'invention est de préférence telle qu'elle ne confère pas de caractère collant à la composition finale. Cette quantité va en général de 0,05 à 20 % en poids et de préférence de 0,1 à 15 % en poids et mieux de 0,5 à 10 % en poids par rapport au poids total de la composition.

Les compositions cosmétiques de l'invention peuvent, en outre, contenir des adjuvants habituels dans le domaine cosmétique, tels que les antioxydants, les parfums, les peptisants de parfums, les filtres, les matières colorantes, les actifs hydrophiles ou lipophiles. La nature des adjuvants et leurs quantités doivent être telles qu'elles ne modifient pas les propriétés de la composition selon l'invention. Les quantités de ces adjuvants sont celles classiquement utilisées dans le domaine cosmétique et par exemple de 0,001 à 10 % du poids total de la composition.

Comme actifs utilisables dans la composition de l'invention, on peut citer par exemple les agents apaisants comme l'allantoïne et le bisabolol ; les eaux florales telles que l'eau de tilleul ou l'eau de bleuet ; l'acide glycyrrhétinique et ses sels ; les antibactériens comme l'octopirox, le triclosan et le triclocarban ; les huiles essentielles ; les vitamines telles que par exemple le rétinol (vitamine A), l'acide ascorbique (vitamine C), le tocophérol (vitamine E), la niacinamide (vitamine PP ou B3), le panthénol (vitamine B5) et leurs dérivés tels que par exemple les esters de ces vitamines (palmitate, acétate, propionate), l'ascorbyl phosphate de magnésium, la vitamine C glycosylée ou acide glucopyranosyl ascorbique (Ascorbyl glucoside) ; les co-enzymes tels que le co-enzyme Q10 ou ubiquinone et le co-enzyme R ou biotine ; les hydrolysats de protéine ; les extraits végétaux et notamment les extraits de plancton ; et leurs mélanges.

La composition de l'invention peut contenir aussi des gélifiants hydrophiles comme par exemple les polymères carboxyvinyliques modifiés ou non, tels que les produits commercialisés sous les dénominations Carbopol (nom INCI : carbomer) et Pemulen (nom INCI : Acrylates/C10-30 akyl acrylate crosspolymer) par la société Noveon ; les polyacrylamides ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination « Hostacerin AMPS » (nom INCI : ammonium polyacryldimethyltaurate) ; les polysaccharides comme la gomme de xanthane, la gomme de guar, les alginates, les celluloses modifiées ou non, les dérivés de fucose comme ceux commercialisés sous les dénominations FUCOGEL 1000 et FUCOGEL 1000 PP par la société Solabia. La quantité de ces polymères peut aller par exemple de 0,05 à 3 % en poids par rapport au poids total de la composition.

Les compositions de l'invention sont particulièrement adaptées au démaquillage de la peau et/ou de la zone oculaire.

Aussi, l'invention a encore pour objet l'utilisation cosmétique de la composition telle que définie ci-dessus pour le démaquillage de la peau et/ou de la zone oculaire.

Les exemples suivants sont donnés à titre d'illustration de l'invention et n'ont pas de caractère limitatif. Toutes les quantités sont données en pourcentage en poids de matière première par rapport au poids total de la composition.

Exemple 1 selon l'invention : Eau démaquillante

- PEG-7 Caprylic/Capric Glycerides        1 %
- Eau        qsp 100 %

Mode opératoire : on mélange le PEG-7 Caprylic/Capric Glycerides et l'eau à température ambiante.

On obtient une eau démaquillante limpide qui permet un très bon démaquillage de la peau, notamment de la peau du visage, et des yeux.

Exemple 2 selon l'invention : Eau démaquillante

- PEG-7 Caprylic/Capric Glycerides        0,5 %
- PEG-6 Caprylic/Capric Glycerides        0,5 %
- Eau        qsp 100 %

Mode opératoire : on mélange le PEG-7 Caprylic/Capric Glycerides, le PEG-6 Caprylic/Capric Glycerides et l'eau à température ambiante.

On obtient une eau démaquillante limpide qui permet un très bon démaquillage de la peau, notamment de la peau du visage, et des yeux.

Exemple 3 selon l'invention : Eau démaquillante

- Conservateurs        0,7 %
- Parfum        0,01 %
- Polysorbate 80        1 %
- PEG-6 Caprylic/Capric Glycerides        1 %
- Poloxamer 184        2 %
- Poloxamer 124        2 %
- PEG-7 Caprylic/Capric Glycerides        1 %
- Eau        qsp 100 %

Mode opératoire : on mélange les tensioactifs (Polysorbate 80, PEG-6 Caprylic/Capric Glycerides, Poloxamer 184, Poloxamer 124 et PEG-7 Caprylic/Capric Glycerides), puis on ajoute dans ce mélange, le parfum, et on mélange le tout avec l'eau contenant les conservateurs.

On obtient une eau démaquillante limpide qui permet un très bon démaquillage de la peau, notamment de la peau du visage, et des yeux.

Comparaison avec d'autres tensioactifs

Exemples 4 à 7 comparatifs : ces exemples comparatifs correspondent à l'exemple 1 dans lequel le PEG-7 Caprylic/ Capric Glycerides a été remplacé par une quantité équivalente d'un autre tensioactif.

| Composition | 1 % de tensioactif | Eau |
|---|---|---|
| Exemple 1 de l'invention | PEG-7 Caprylic/Capric Glycerides | Qsp 100 % |
| Exemple 4 comparatif | Polysorbate 80 | Qsp 100 % |
| Exemple 5 comparatif | PEG-6 Caprylic/Capric Glycerides | Qsp 100 % |
| Exemple 6 comparatif | Poloxamer 184 | Qsp 100 % |
| Exemple 7 comparatif | Poloxamer 124 | Qsp 100 % |

Un test comparatif portant sur le pouvoir démaquillant de l'exemple 1 de l'invention et des exemples comparatifs a été réalisé selon le protocole décrit ci-dessous.

1. Matériel

- Composition démaquillante
- Savon de Marseille
- 1 cache découpé dans une fiche bristol de 4x4 cm + 1 feutre LUMICOLOR permanent S
- Colorimètre CR300 (mesure de la colorimétrie en L.a.b)
- Fond de teint sans transfert Air Wear LSF 12
- 8 petits verres de montre + des petites spatules
- 1 balance de précision
- 1 chronomètre

2. Protocole

a- Tracer sur la peau des bras, 4 zones (2 par bras) à l'aide du feutre et du cache
b- Nettoyer la peau au savon, rincer et sécher (les marques de feutre sont alors atténuées pour éviter tout transfert de feutre dans le fond de teint au moment de l'application).
c-Mesurer par colorimétrie la peau nue : 3 mesures de L,a,b par zone
d- Appliquer dans les 4 zones délimitées, de façon homogène, 16mg+/-0,05mg de fond de teint et attendre 30 minutes
e- Mesurer par colorimétrie la peau avec fond de teint : 3 mesures de L,a,b par zone
f- Peser dans les verres de montre 4 x 208 mg+/-1 mg de produit démaquillant à tester
g- Démaquiller la zone au doigt par mouvement circulaire pendant 10 secondes puis rincer à l'eau du robinet (tiède ; dureté non contrôlée) en effleurant légèrement avec les doigts de la main disponible. Faire les 4 zones sans attendre.
h- Tamponner avec un mouchoir en papier type kleenex et attendre 15 minutes (sensation de peau sèche)
i- Mesurer par colorimétrie la peau démaquillée : 3 mesures de L,a,b par zone

3. Calcul du pourcentage de démaquillage

a- Ecart colorimétrique de la peau maquillée par rapport à la peau nue = ΔEmax :

$$\Delta E\ max = \sqrt{(\Delta a_1{}^2 + \Delta b_1{}^2 + \Delta L_1{}^2)}$$

avec

$\Delta a_1$ = a peau nue - a peau FdT
$\Delta b_1$ = b peau nue - b peau FdT
$\Delta L_1$ = L peau nue - L peau FdT

b- Pour un produit démaquillant : Ecart colorimétrique de la peau démaquillée par rapport à la peau maquillée = ΔE pour chaque zone :

$$\Delta E = \sqrt{(\Delta a_2^2 + \Delta b_2^2 + \Delta L_2^2)}$$

avec

$\Delta a_2$ = a peau démaq - a peau FdT
$\Delta b_2$ = b peau démaq - b peau FdT
$\Delta L_2$ = L peau démaq - L peau FdT

Le pourcentage de démaquillage moyen correspond à la moyenne des 4 valeurs de $(\Delta E / \Delta E max)$ *100 pour un produit démaquillant donné.
Le tableau ci-dessous donne les résultats du pourcentage de démaquillage (pourcentage de maquillage enlevé) pour chaque composition.

| Composition | Pourcentage de démaquillage |
|---|---|
| Exemple 1 selon l'invention | 71 % |
| Exemple 4 comparatif | 27 % |
| Exemple 5 comparatif | 47 % |
| Exemple 6 comparatif | 32 % |
| Exemple 7 comparatif | 22 % |

Ce tableau montre que la composition selon l'invention présente une efficacité de démaquillage bien plus importante que les compositions des exemples comparatifs, même par rapport à la composition de l'exemple 5 comparatif qui contient un tensioactif (PEG-6 Caprylic/Capric Glycerides) ayant une structure analogue à celle du PEG-7 Caprylic/Capric Glycerides.

Comparaison avec des compositions contenant des huiles démaquillantes

L'efficacité démaquillante de l'exemple 1 selon l'invention a été évaluée comparativement à des laits démaquillants contenant des huiles.
Les laits démaquillants avaient les compositions suivantes :

Exemple 8 comparatif :

| Phase A : | |
|---|---|
| Eau | qsp 100 % |
| Conservateurs | 0,2% |
| Glycerine | 5 % |
| Tensioactifs nettoyants (Disodium Cocoamphodiacetate/ Sodium Laureth Sulfate) | 1,5 % |
| Gomme de xanthane | 0,1 % |
| Phase B : | |
| Huile de vaseline | 12 % |
| Palmitate d'isopropyle | 7,5 % |
| Alcool cétéarylique | 0,4 % |
| Ceteareth-30 | 0,1 % |
| Carbomer | 0,6 % |
| Phase C : | |
| Eau | 5 % |

(suite)

*Phase C :*
NaOH                                                                                    qsp pH 6,5

Exemple 9 comparatif :

| | |
|---|---|
| *Phase A :* | |
| Eau | qsp 100 % |
| Conservateurs | 0,2 % |
| Glycérine | 3 % |
| Tensioactifs nettoyants (Disodium Cocoamphodiacetate/ Sodium Laureth Sulfate) | 1,5 % |
| *Phase B :* | |
| Palmitate d'éthylhexyle | 15 % |
| Pemulen TR-2 | 0,15 % |
| *Phase C :* | |
| Eau | 5 % |
| NaOH | qsp pH 6,5 |

Le pouvoir démaquillant pour les yeux a été évalué selon le même test que celui décrit pour les exemples comparatifs 4 à 7.

| Composition | Pourcentage de démaquillage |
|---|---|
| Exemple 1 selon l'invention | 71 % |
| Exemple 8 comparatif | 19 % |
| Exemple 9 comparatif | 26 % |

Ces résultats montrent que la composition selon l'invention a une efficacité de démaquillage bien meilleure que des laits démaquillants contenant respectivement 19,5% et 15% d'huiles démaquillantes, qui sont les taux habituellement utilisés dans les laits démaquillants.

Tests sur panel

Par ailleurs, deux tests in vivo ont été réalisés avec la composition de l'exemple 3 selon l'invention, sur un panel de 50 personnes pendant 4 semaines. Dans le premier test, la composition a été utilisée en tant de démaquillant des yeux, et dans le second test, la composition a été utilisée en tant de démaquillant du visage et des yeux.
Les résultats sont les suivants :

1) Test n °1 : efficacité démaquillante de l'exemple 3 en tant que démaquillant des yeux

Protocole :

Nombre de sujets : 50
Critères d'inclusion : Sujets de tous types de peau, dont 50% utilisant un maquillage Waterproof, 20 % ayant les yeux sensibles, 20 % portant des lentilles, 10 % ayant les yeux normaux.
Durée du traitement : 4 semaines
Zone d'application : yeux
Schéma expérimental : démaquillage une fois par jour

Résultats (les % correspondent aux pourcentages de personnes ayant noté le critère)

| Critère après 4 semaines d'utilisation | % de personnes ayant noté le critère |
|---|---|
| Critères cosmétiques | |
| Produit doux pour les yeux | 98.0% |
| Produit non gras | 95.9% |
| Produit non collant | 98.0% |
| Laisse les yeux confortable | 87.8% |
| Critères d'efficacité | |
| Elimine le maquillage waterproof | 78.6% |
| Démaquille parfaitement les yeux | 89.8% |
| Démaquille en douceur | 98.0% |
| Démaquille en un seul geste | 49.0% |
| Démaquille facilement | 73.5% |
| Décongestionne les paupières fatiguées | 59.2% |
| Ne dessèche pas la peau du contour de l'oeil | 91.8% |
| Laisse une sensation de fraîcheur sur les yeux | 85.7% |

Les personnes qui ont utilisé la composition selon l'invention ont apprécié tous les critères cosmétiques (87% à 98%), en particulier la douceur de la composition pour les yeux, et l'aspect non gras et non collant de la composition.
Par ailleurs, les personnes qui ont utilisé la composition selon l'invention ont apprécié les critères d'efficacité (49% à 98%), en particulier le démaquillage en douceur, le non desséchement de la peau du contour de l'oeil, le démaquillage parfait des yeux et la sensation de fraîcheur sur les yeux. Environ la moitié des sujets ont trouvé que la composition démaquillait en un seul geste, ce qui n'est pas courant dans le démaquillage. Globalement, la composition selon l'invention a obtenu une très bonne appréciation de son efficacité sur la zone sensible de la sphère oculaire.

2) Test n°2 : efficacité démaquillante de l'exemple 3 en tant que démaquillant du visage et des yeux

Protocole :

Nombre de sujets : 42 personnes
Critères d'inclusion : Sujets ayant la peau normale à mixte, utilisant quotidiennement des produit de maquillage visage et yeux, dont un minimum de dix utilisatrices de mascaras waterproof.
Durée du traitement : une semaine
Zone d'application : Visage et yeux
Schéma expérimental : démaquillage une fois par jour, le soir

Résultats :

| Satisfaction globale | Nombre de personnes |
|---|---|
| Très satisfaite | 11 sur 28 |
| Satisfaite | 8 sur 28 |
| Plutôt satisfaite | 3 sur 28 |
| Plutôt insatisfaite | 3 sur 28 |
| Insatisfaite | 3 sur 28 |

(suite)

| Satisfaction globale | Nombre de personnes |
|---|---|
| Très insatisfaite | 0 |
| Note moyenne globale sur 10 | 7,8 sur 10 |

Par ailleurs, le confort a été évalué sur le visage et sur les yeux :

- pour le confort sur le visage, 25 personnes sur 28 ont trouvé que le confort à l'application était très bon à assez bon, et 21 personnes sur 28 ont trouvé que le confort après l'application était très bon à assez bon.

- pour le confort sur les yeux, pour le mascara classique, 16 personnes sur 19 ont trouvé que le confort à l'application était très bon à assez bon, et 17 personnes sur 19 ont trouvé que le confort après l'application était très bon à assez bon, et pour le mascara Waterproof, 10 personnes sur 13 ont trouvé que le confort à l'application était très bon à assez bon, et 10 personnes sur 13 ont trouvé que la confort après l'application était très bon à assez bon,

En outre, l'efficacité démaquillante a été également évaluée :

- sur le visage, 23 personnes sur 28 ont trouvé que la composition démaquillait très rapidement à assez rapidement,
- sur les yeux, pour le mascara classique, 17 personnes sur 19 ont trouvé que la composition démaquillait très rapidement à assez rapidement, et pour le mascara Waterproof, 10 personnes sur 13 ont trouvé que la composition démaquillait très rapidement à assez rapidement.

Ces résultats montrent qu'une majorité de personnes ayant utilisé la composition de l'invention a été satisfaite de son efficacité et de son confort d'utilisation, et ils confirment l'excellente efficacité démaquillante sur le visage et les yeux de la composition selon l'invention.

## Revendications

1. Composition cosmétique aqueuse démaquillante, **caractérisée en ce qu'**elle contient un dérivé de polyéthylène glycol et d'un mélange de glycérides d'acide caprylique et d'acide caprique, comportant 7 groupes d'oxyde d'éthylène, en une quantité inférieure à 10 % en poids par rapport au poids total de la composition.

2. Composition selon la revendication 1, **caractérisée en ce qu'**elle comprend au moins 55 % en poids d'eau par rapport au poids total de la composition.

3. Composition selon la revendication 1 ou 2, **caractérisée en ce que** le dérivé de polyéthylène glycol et du mélange de glycérides d'acide caprylique et d'acide caprique, comportant 7 groupes d'oxyde d'éthylène, est présent en une quantité allant de 0,01 à 10 % en poids, de préférence de 0,05 à 10 % en poids par rapport au poids total de la composition.

4. Composition selon l'une quelconque des revendications précédentes, **caractérisée en ce qu'**elle contient, en outre, un ou plusieurs tensioactifs choisis parmi les tensioactifs non ioniques, les tensioactifs anioniques, les tensioactifs amphotères ou zwitterioniques et leurs mélanges.

5. Composition selon la revendication précédente, **caractérisée en ce que** le ou les tensioactifs sont choisis parmi les polymères blocs oxyéthylénés oxypropylénés, les esters de sorbitan polyoxyéthylénés, les dérivés de polyéthylène glycol d'un mélange de glycérides d'acide caprylique et d'acide caprique, ayant de 6 ou 8 groupes d'oxyde d'éthylène, et leurs mélanges.

6. Composition selon l'une quelconque des revendications précédentes, **caractérisée en ce qu'**elle contient, en outre, un dérivé de polyéthylène glycol d'un mélange de glycérides d'acide caprylique et d'acide caprique, ayant de 6 groupes d'oxyde d'éthylène.

7. Composition selon l'une quelconque des revendications précédentes, **caractérisée en ce que** la quantité totale de tensioactif(s) va de 0,01 à 12 % en poids par rapport au poids total de la composition.

8. Composition selon l'une quelconque des revendications précédentes, **caractérisée en ce qu'**elle constitue une eau de démaquillage de la peau et/ou de la zone oculaire.

9. Utilisation cosmétique de la composition selon l'une quelconque des revendications 1 à 7, pour le démaquillage de la peau et/ou de la zone oculaire.

10. Utilisation cosmétique d'un dérivé de polyéthylène glycol et d'un mélange de glycérides d'acide caprylique et d'acide caprique, comportant 7 groupes d'oxyde d'éthylène, en une quantité inférieure à 10 % en poids par rapport au poids total de la composition, pour le démaquillage de la peau ou de la zone oculaire.

**Revendications modifiées conformément à la règle 86(2) CBE.**

1. Composition cosmétique aqueuse démaquillante, **caractérisée en ce qu'**elle contient un dérivé de polyéthylène glycol et d'un mélange de glycérides d'acide caprylique et d'acide caprique, comportant 7 groupes d'oxyde d'éthylène (PEG-7 Caprylic/Capric Glycerides), en une quantité inférieure à 10 % en poids par rapport au poids total de la composition, la composition étant exempte d'huile.

2. Composition selon la revendication 1, **caractérisée en ce qu'**elle comprend au moins 55 % en poids d'eau par rapport au poids total de la composition.

3. Composition selon la revendication 1 ou 2, **caractérisée en ce que** le dérivé de polyéthylène glycol et du mélange de glycérides d'acide caprylique et d'acide caprique, comportant 7 groupes d'oxyde d'éthylène, est présent en une quantité allant de 0,01 à 10 % en poids, de préférence de 0,05 à 10 % en poids par rapport au poids total de la composition.

4. Composition selon l'une quelconque des revendications précédentes, **caractérisée en ce qu'**elle contient, en outre, un ou plusieurs tensioactifs choisis parmi les tensioactifs non ioniques, les tensioactifs anioniques, les tensioactifs amphotères ou zwitterioniques et leurs mélanges.

5. Composition selon la revendication précédente, **caractérisée en ce que** le ou les tensioactifs sont choisis parmi les polymères blocs oxyéthylénés oxypropylénés, les esters de sorbitan polyoxyéthylénés, les dérivés de polyéthylène glycol d'un mélange de glycérides d'acide caprylique et d'acide caprique, ayant de 6 ou 8 groupes d'oxyde d'éthylène, et leurs mélanges.

6. Composition selon l'une quelconque des revendications précédentes, **caractérisée en ce qu'**elle contient, en outre, un dérivé de polyéthylène glycol d'un mélange de glycérides d'acide caprylique et d'acide caprique, ayant de 6 groupes d'oxyde d'éthylène.

7. Composition selon l'une quelconque des revendications précédentes, **caractérisée en ce que** la quantité totale de tensioactif(s) va de 0,01 à 12 % en poids par rapport au poids total de la composition.

8. Composition selon l'une quelconque des revendications précédentes, **caractérisée en ce qu'**elle constitue une eau de démaquillage de la peau et/ou de la zone oculaire.

9. Utilisation cosmétique de la composition selon l'une quelconque des revendications 1 à 7, pour le démaquillage de la peau et/ou de la zone oculaire.

10. Utilisation cosmétique d'un dérivé de polyéthylène glycol et d'un mélange de glycérides d'acide caprylique et d'acide caprique, comportant 7 groupes d'oxyde d'éthylène (PEG-7 Caprylic/Capric Glycerides), en une quantité inférieure à 10 % en poids par rapport au poids total de la composition, pour le démaquillage de la peau ou de la zone oculaire, la composition étant exempte d'huile.

**Office européen
des brevets**

**RAPPORT DE RECHERCHE EUROPEENNE**

Numéro de la demande

EP 06 11 9306

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | US 5 942 238 A (MCATEE ET AL.) 24 août 1999 (1999-08-24) * revendications 1-6; exemples 1,8 * | 1-10 | INV. A61K8/86 A61Q1/14 |
| A | US 6 113 892 A (NEWELL ET AL.) 5 septembre 2000 (2000-09-05) * revendications 1,3,4; exemples 1,3-7 * | 1-10 | |
| A | RENAE CANTERBERY PEPE ET AL.: "International Cosmetic Ingredient Dictionary and Handbook" 2002, COSMETIC, TOILETRY AND FRAGRANCE ASSOCIATION , WASHINGTON D.C. , XP002386122 * page 1120 * * page 1142 * | 1-10 | |

**DOMAINES TECHNIQUES RECHERCHES (IPC)**

A61K

Le présent rapport a été établi pour toutes les revendications

| Lieu de la recherche | Date d'achèvement de la recherche | Examinateur |
|---|---|---|
| Berlin | 15 décembre 2006 | ALVAREZ ALVAREZ, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

&  : membre de la même famille, document correspondant

**ANNEXE AU RAPPORT DE RECHERCHE EUROPEENNE
RELATIF A LA DEMANDE DE BREVET EUROPEEN NO.**

EP 06 11 9306

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

15-12-2006

| Document brevet cité au rapport de recherche | | Date de publication | Membre(s) de la famille de brevet(s) | | Date de publication |
|---|---|---|---|---|---|
| US 5942238 | A | 24-08-1999 | AU | 6033498 A | 18-08-1998 |
| | | | CA | 2278752 A1 | 30-07-1998 |
| | | | CN | 1244788 A | 16-02-2000 |
| | | | EP | 0981317 A1 | 01-03-2000 |
| | | | JP | 2002504089 T | 05-02-2002 |
| | | | WO | 9832416 A1 | 30-07-1998 |
| US 6113892 | A | 05-09-2000 | AUCUN | | |

EPO FORM P0460

Pour tout renseignement concernant cette annexe : voir Journal Officiel de l'Office européen des brevets, No.12/82